# EUROPEAN PATENT APPLICATION

(11) **EP 1 437 337 A1**
(43) Date of publication of application: **14.07.2004**
(21) Application number: 02779897.4
(22) Date of filing: 26.09.2002
(51) Int. Cl.: C07C 31/20, C07C 29/86, C07C 29/80

(54) **1,3-BUTYLENE GLYCOL AND PROCESS FOR PRODUCING THE SAME**

(30) Priority: 26.09.2001 JP 2001294073
(71) Applicant: Kyowa Yuka Co., Ltd., Tokyo 100-8185 (JP)
(72) Inventor: MIZUTANI, Satoru,, Yokkaichi-shi, Mie 512-8048 (JP); HISAMURA, Koji, c/o Kyowa Yuka Co., Ltd., Yokkaichi-shi, Mie 510-8502 (JP); KINOSHITA, Hirotaka, c/o Kyowa Yuka Co., Ltd., Yokkaichi-shi, Mie 510-8502 (JP)
(74) Representative: Casalonga, Axel
(86) International application number: PCT/JP2002/009909
(87) International publication number: WO 2003/027053

(57) **Abstract**

The present invention provides the following [1] to [3], etc.
[1] 1, 3-Butylene glycol where, in a gas chromatographic analysis under a specific condition, the sum of area values of peaks appearing during the specific relative retention time to the area value of the peak of 1,3-butylene glycol is not more than 0.025%.
[2] 1,3-Butylene glycol where, in a gas chromatographic analysis under a specific condition, the sum of area values of peaks of compounds having a hydroxyl group and an ether bond and having a molecular weight of 188 to the area value of the peak of 1,3-butylene glycol is not more than 0.025%.
[3] 1,3-Butylene glycol where, in a gas chromatographic analysis under a specific condition, the sum of area values of peaks of 2-butoxy-6-methyl-4-tetrahydropyranol to the area value of the peak of 1, 3-butylene glycol is not more than 0.025%.

## Description

### Technical Field

The present invention relates to 1,3-butylene glycol which is useful as a material for synthetic resins, a material for surfactants, a solvent, an antifreeze, a material for cosmetics, etc.

### Background of the Invention

1,3-Butylene glycol has properties such as non-volatility, low toxicity and hygroscopicity and, therefore, it is useful for amoisturizer in cosmetics, and the like. When 1, 3-butylene glycol is utilized as a moisturizer in cosmetics, it has been desired to use 1,3-butylene glycol having little smell.

With regard to a means for preparing 1, 3-butylene glycol having little smell, Japanese Unexamined Patent Application No. 258192/95, for example, discloses a method where, in conducting distillation for the removal of high-boiling substances, distillation is carried out after addition of a compound like sodium hydroxide. Moreover, WO 00/07969 discloses a method where an alkali metal base is added to crude 1,3-butylene glycol, where high-boiling substances are removed, followed by treatment with heat, 1,3-butylene glycol is distilled so as to separate the alkali metal compound and the high-boiling substances as a residue, and then low-boiling substances are distilled from the fraction of 1,3-butylene glycol. However, even the 1,3-butylene glycol prepared by any of the above-mentioned methods still has a smell and, in addition, the smell increases upon storage whereby it is not satisfactory in practical use.

### Disclosure of the Invention

An object of the present invention is to provide 1,3-butylene glycol having little smell.

The present invention provides the following [1] to [9] , etc.
[1] 1,3-Butylene glycol where, in a gas chromatographic analysis under a specific condition, the sum of area values of peaks appearing during the specific relative retention time to the area value of the peak of 1,3-butylene glycol is not more than 0.025% (hereinafter, the above 1,3-butylene glycol may be referred to as 1,3-butylene glycol A).
[2] 1, 3-Butylene glycol where, in a gas chromatographic analysis under a specific condition, the sum of area values of peaks of compounds having a hydroxyl group and an ether bond and having a molecular weight of 188 to the area value of the peak of 1, 3-butylene glycol is notmore than 0.025% (hereinafter, the above 1, 3-butylene glycol may be referred to as 1, 3-butylene glycol B).
[3] 1,3-Butylene glycol where, in a gas chromatographic analysis under a specific condition, the sum of area values of peaks of 2-butoxy-6-methyl-4-tetrahydropyranol to the area value of the peak of 1, 3-butylene glycol is not more than 0.025% (hereinafter, the above 1,3-butylene glycol may be referred to as 1,3-butylene glycol C).
[4] ] 1, 3-Butylene glycol where, in a gas chromatographic analysis under a specific condition, the sum of area values of peaks appearing during the specific relative retention time to the sum of area values of the peaks appearing during the relative retention time of 0 to 1.85 when the retention time of 1,3-butylene glycol is defined as 1 is not more than 0.025% (hereinafter, the above 1,3-butylene glycol may be referred to as 1,3-butylene glycol D).
[5] The 1,3-butylene glycol according to [1] or [4], wherein the peaks appearing during the specific relative retention time are the peaks having an apex during the relative retention time of 1.4 to 1.48 or during the relative retention time of 1.7 to 1. 8 when the retention time of 1, 3-butylene glycol is defined as 1.
[6] The 1,3-butylene glycol according to any of [1] to [5], wherein the gas chromatographic analysis under a specific condition is a gas chromatographic analysis using a column using polyethylene glycol in a liquid phase.
[7] The 1,3-butylene glycol according to any of [1] to [6], wherein the gas chromatographic analysis under a specific condition is carried out under the following gas chromatographic analysis condition.
   Condition for the gas chromatographic analysis:
   Apparatus: HP 5890 Series II manufactured by Hewlett-Packard
   Column: Capillary column DB-WAX (length: 30 m; inner diameter: 0.25 mm; membrane thickness: 0.25 µm) manufactured by J & W Scientific

   Temperature-rising condition: After the retention at 130°C for 10 minutes, the temperature is raised up to 140°C at the rate of 1°C/minute and retained at 140°C.
   Sample-introducing part: Split sample introduction method; temperature: 250°C
   Flow rate of gas at the split (carrier gas: nitrogen): 67 ml/minute
   Flow rate of gas through the column (carrier gas: nitrogen): 0.86 ml/minute
   Detector: hydrogen flame ionization detector (FID); temperature: 250°C
[8] A process for producing 1,3-butylene glycol comprising a step of mixing crude 1, 3-butylene glycol with water and an organic solvent followed by separation of an aqueous layer and an organic layer to obtain an aqueous layer containing 1,3-butylene glycol.
[9] The process for producing 1,3-butylene glycol according to [8], wherein the aqueous layer containing 1,3-butylene glycol is subjected to distillation.

Hereinafter, 1,3-butylene glycol A, B, C and D may be together referred to as 1,3-butylene glycol of the present invention in some cases.

The crude 1,3-butylene glycol used as a material in the process for producing of the present invention includes, for example, 1,3-butylene glycol having a smell or 1,3-butylene glycol where the smell increases with a lapse of time.

With regard to the crude 1,3-butylene glycol, 1,3-butylene glycol where the sum of area values of peaks appearing during the specific relative retention time to the area value of the peak of 1,3-butylene glycol in a gas chromatographic analysis under a specific condition, is not less than 0. 03% is preferably used and 1, 3-butylene glycol where it is not less than 0.04% is more preferably used.

With regard to the crude 1,3-butylene glycol, 1,3-butylene glycol where the sum of area values of peaks of compounds having a hydroxyl group and an ether bond and having a molecular weight of 188 to the area value of the peak of 1,3-butylene glycol in a gas chromatographic analysis under a specific condition, is not less than 0 .03% is preferably used and 1,3-butylene glycol where it is not less than 0.04% is more preferably used.

With regard to the crude 1,3-butylene glycol, 1,3-butylene glycol where the sum of area values of peaks of 2-butoxy-6-methyl-4-tetrahydropyranol to the area value of the peak of 1,3-butylene glycol in a gas chromatographic analysis under a specific condition, is not less than 0.03% is preferably used and 1,3-butylene glycol where it is not less than 0.04% is more preferably used.

With regard to the crude 1,3-butylene glycol, 1,3-butylene glycol where the sum of area values of peaks appearing during the specific relative retention time to the sum of area values of the peaks appearing during the relative retention time of 0 to 1.85 when the retention time of 1,3-butylene glycol is defined as 1 in a gas chromatographic analysis under a specific condition, is not less than 0.03% is preferably used and 1,3-butylene glycol where it is not less than 0.04% is more preferably used.

There is no particular limitation for the process for producing the crude 1,3-butylene glycol and, for example, a crude 1,3-butylene glycol may be produced by a publicly known method (refer to Japanese Examined Patent Application No. 80139/91, Japanese Unexamined Patent Application No. 158129/95, etc.)

It is also possible to use a product prepared by such a manner that ethanol which is a by-product is removed by distillation from a reaction product obtained by reduction of acetaldol with hydrogen or a product prepared by such as manner that the fraction after removal of ethanol is further subjected to one or more known purifying step(s) such as distillation or a step of heating after addition of an alkaline metal compound (such as sodium hydroxide andpotassiumhydroxide) (WO 00/07969 ) as a crude 1,3-butylene glycol. The crude 1,3-butylene glycol may be also available as a commercial product.

Next, the process for producing 1,3-butylene glycol of present invention is described.

The 1,3-butylene glycol of the present invention may be prepared, for example, by mixing crude 1, 3-butylene glycol with water and an organic solvent the solution is separated into an aqueous layer and an organic layer to give the aqueous layer containing 1, 3-butylene glycol by removing the water therefrom. Examples of the organic solvent used are aromatic hydrocarbons such as toluene and xylene; ketones such as methyl isobutyl ketone; an aliphatic hydrocarbon such as hexane and heptane; esters such as ethyl acetate and butyl acetate; ethers such as diethyl ether and dibutyl ether; and an organic chloride such as methylene chloride and chloroform. Among them, ketones are preferred and methyl isobutyl ketone is more preferred. The above-mentioned organic solvent may be used solely or two or more thereof may be selected and used after mixing in any ratio. The amount of the organic solvent used to 100 parts by weight of crude 1, 3-butylene glycol is preferably 10 to 300 parts by weight and, more preferably, 20 to 200 parts by weight.

Amount of water to be used to 100 parts by weight is preferably 20 to 400 parts by weight and, more preferably, 40 to 200 parts by weight.

There is no particular limitation to the order of mixing of crude 1,3-butylene glycol, water and an organic solvent.

Although there is no particular limitation to the temperature when crude 1, 3-butylene glycol is mixed with water and an organic solvent, the temperature between 5 to 80°C is preferred and that between 10 to 60°C is more preferred.

Mixing of crude 1,3-butylene glycol with water and an organic solvent may be carried out, for example, by a batch system or a continuous system.

An example for a mode of carrying out the batch system is that crude 1,3-butylene glycol, water and an organic solvent are placed in a mixing vessel, stirred preferably for 10 seconds to 2 hours and allowed to stand preferably for 1 minute to 2 hours to separate into phases whereupon an aqueous phase containing 1, 3-butylene glycol is obtained. It is alsopossible to repeat an operation where an organic solvent is further added to the resulting aqueous layer containing 1,3-butylene glycol to separate into phases so that an aqueous layer containing 1,3-butylene glycol is prepared. With regard to the time for the repetition, one to three times is preferred. In that case, it is preferred that the amount of the organic solvent to be added per operation is 10 to 300 parts by weight to 100 parts by weight of the crude 1,3-butylene glycol.

With regard to an apparatus used for the continuous system, it is possible to use an apparatus which is commonly used for a continuous extraction such as a combination of mixer and settler, spraying tower, packed tower, shelf-plate tower, etc. The use of a packed tower or a shelf-plate tower where the theoretical plates are 3 or more is particularly preferred.

It is preferred that the process for of the present invention contains a step where crude 1,3-butylene glycol is mixed with water and an organic solvent and subjected to a phase separation into an aqueous layer and an organic layer to give an aqueous layer containing 1, 3-butylene glycol and a step where the aqueous layer is subjected to distillation. The distillation tower used for subjecting the aqueous layer to distillation is, for example, porous plate tower, bubble cap tower or packed tower and, among them, a packed tower where the theoretical plate numbers are 7 to 40 is preferred. With regard to a distilling tower, one tower may be used or two or more thereof may be used. With regard to the condition for the distillation, pressure of the top of the distilling tower is preferably 5 to 20 kPa and, more preferably, 5 to 10 kPa while temperature of the bottom of the distilling tower is preferably 120 to 160°C and, more preferably, 135 to 155°C. A specific example for the embodiment is a method where an aqueous layer containing 1,3-butylene glycol is continuously supplied from the top of the distilling tower, then a fraction containing much water is continuously taken out from the top and, at the same time, 1,3-butylene glycol is continuously taken out from the bottom of the tower. 1, 3-Butylene glycol which is obtained from the bottom of the tower may be further subjected to a known purifying method such as distillation if necessary.

In 1,3-butylene glycol of the present invention, a gas chromatographic analysis therefor under a specific condition is preferably a gas chromatographic analysis using a column where polyethylene glycol is used in a liquid phase and, more preferably, a gas chromatographic analysis carried out under the following analytical condition.

Condition for the gas chromatographic analysis:
Apparatus: HP 5890 Series II manufactured by Hewlett-Packard
Column: Capillary column DB-WAX (length: 30 m; inner diameter: 0.25 mm; membrane thickness: 0.25 µm) manufactured by J & W Scientific
Temperature-rising condition: After retaining at 130°C for 10 minutes, temperature is raised up to 140°C at the rate of 1°C/minute and retained at 140°C.

Sample-introducing part: Split sample introduction method; temperature: 250°C
Flow rate of gas at the split (carrier gas: nitrogen): 67 ml/minute
Flow rate of gas through the column (carrier gas: nitrogen): 0.86 ml/minute
Detector: hydrogen flame ionization detector (FID); temperature: 250°C

Preparation of the sample for analysis: A mixture of 1 part by weight of the sample and 1 part by weight of distilled water is infused in an amount of 1 µl.

Peaks appearing during the specific relative retention time in a gas chromatographic analysis under a specific condition in 1,3-butylene glycol A and 1,3-butylene glycol D mean the the peak having an apex of the peak at the retention time within a range of 1.4 to 1.48 or, preferably, 1.4 to 1.475 (hereinafter, the peak having the apex within this range may be referred to as the peak A) or the peak having an apex of the peak at the retention time within a range of 1.7 to 1.8 or, preferably, 1.72 to 1.78 (hereinafter, the peak having the apex within this range may be referred to as the peak B) when the retention time of 1,3-butylene glycol is defined as 1.

With regard to compounds corresponding to the peak A and the peak B, compounds having a hydroxyl group and an ether bond and having a molecular weight of 188 may be exemplified and, to be more specific, 2-butoxy-6-methyl-4-tetrahydropyranol, etc. may be exemplified.

In 1, 3-butylene glycol A, the sum of area values of peaks appearing during the specific relative retention time in a gas chromatographic analysis under a specific condition to area value of the peak of 1,3-butylene glycol is preferably not more than 0.020%, more preferably not more than 0.015% and, still more preferably, not more than 0.010%.

In 1, 3-butylene glycol B, the sum of area values of peaks in a gas chromatographic analysis under a specific condition for compounds having a hydroxyl group and an ether bond and having a molecular weight of 188 to area value of the peak of 1,3-butylene glycol is preferably not more than 0.020%, more preferably not more than 0.015% and, still more preferably, not more than 0.010%.

In 1,3-butylene glycol C, the sum of area values of peaks in a gas chromatographic analysis under a specific condition for 2-butoxy-6-methyl-4-tetrahydropyranol to area value of the peak of 1, 3-butylene glycol is preferably not more than 0.020%, more preferably not more than 0.015% and, still more preferably, not more than 0.010%.

In 1, 3-butylene glycol D, the sum of area values of peaks appearing during the specific relative retention time in a gas chromatographic analysis under a specific condition to the sum of area values of the peaks appearing during the relative retention time of 0 to 1.85 when the retention time of 1,3-butylene glycol is defined as 1 is preferably not more than 0.020%, more preferably not more than 0.015% and, still more preferably, not more than 0.010%.

1,3-Butylene glycol of the present invention has little smell and the smell rarely increases with a lapse of time.

### Test Example 1

Evaluation of smell regarding 1,3-butylene glycol prepared in Examples 1 to 3, was carried out according to the following method.

Preparation of samples used as the standards for the smell: Using a 10% aqueous solution (referred to as "original solution" in the following Table 1) of 1, 3-butylene glycol manufactured by Kyowa Hakko Kogyo, each 100 g of five standard smell samples with different intensity of smell was different were prepared according to the following Table 1 and placed in a 200-ml wide-mouth glass bottle.

**Table 1**

| Level of Smell | 1 | 2 | 3 | 4 | 5 |
|---|---|---|---|---|---|
| Composition Ratio: Original solution/distilled water (w/w) | 1/20 | 1/50 | 1/100 | 1/200 | 1/400 |
| Level of Smell 1: smell is noted 2: between 1 and 3 3: weak smell is noted 4: between 3 and 5 5: smell is not noted | | | | | |

Method of Evaluation of Smell: A 10% aqueous solution (40 g) of the sample was placed in a 100-ml wide-mouth glass bottle, covered and vigorously stirred for 1 minute. The cover was opened and the smell was checked and compared with the standard smell sample whereupon level of the smell of the sample was determined.

Result of Evaluation: In accordance with the above-mentioned method of evaluation, evaluation of smell was carried out for 1,3-butylene glycol prepared in Examples 1 to 3 whereupon all of them were in level 5.

### Test Example 2

Each 100 g of 1,3-butylene glycol prepared in Examples 1 to 3 were placed in a 280-ml container made of stainless steel (made of SUS 304), made into a nitrogen atmosphere, tightly sealed and stored in a constant-temperature vessel of 40°C. Evaluation of smell of each 1, 3-butylene glycol after 3 months was carried out in the same manner as in Test Example 1. With regard to the smell of each 1, 3-butylene glycol after 3 months, all of the products of Examples 1 to 3 were in level 5. Thus, there was no increase in smell for all of 1, 3-butylene glycol prepared in Examples 1 to 3.

### Brief Description of the Drawings

Fig. 1 is a chart of a gas chromatographic analysis of 1,3-butylene glycol prepared in Example 1.
Fig. 2 is a chart of a gas chromatographic analysis of 1,3-butylene glycol prepared in Comparative Example 1.

In the drawings, symbols 1 and 2 show peaks of 1, 3-butylene glycol; symbol 3 shows the peak A; and symbol 4 shows the peak B.

### Best Mode for Carrying Out the Invention

With regardto 1,3-butylene glycol to be used as amaterial, 1,3-butylene glycol manufactured by Kyowa Hakko Kogyo (Product Name: 1,3-Butylene Glycol) was used [hereinafter, this may be referred to as 1, 3-butylene glycol manufactured by Kyowa Hakko Kogyo].

Incidentally, each 1,3-butylene glycol prepared in Examples and Comparative Examples was subjected to a gas chromatographic analysis under the above-mentioned gas chromatographic (GC) analytical condition.

### Example 1

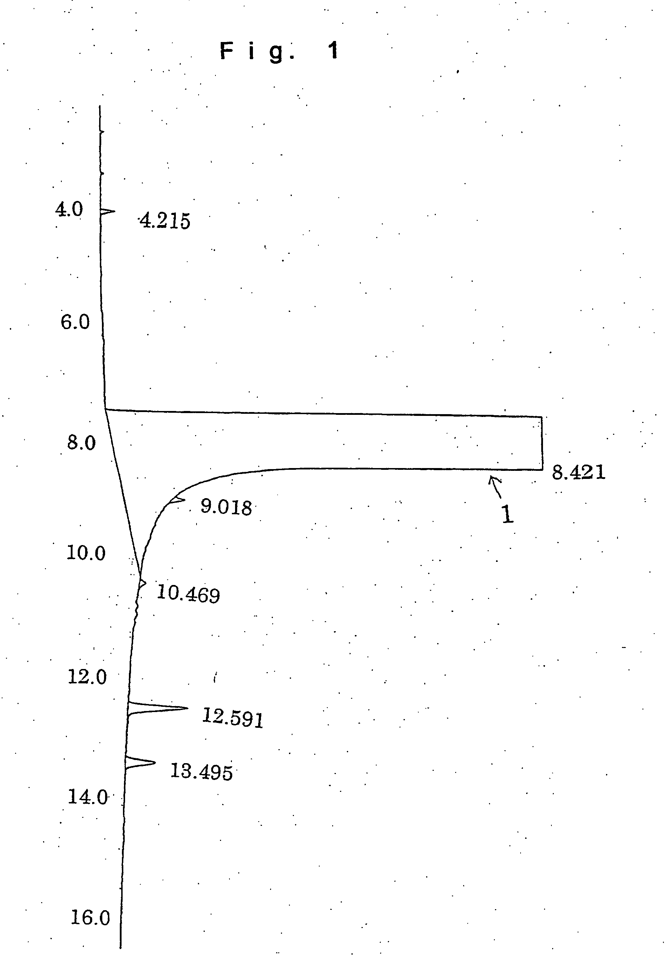

1,3-Butylene glycol manufactured by Kyowa Hakko Kogyo (100 g), 100 g of water and 100 g of methyl isobutyl ketone (hereinafter, may be referred to as MIBK) were charged in to a 500-ml separable flask, stirred at 500 rpm for 10 minutes and allowed to stand for 5 minutes to separate into phases of an aqueous layer and an organic layer. MIBK (100 g) was further added to the separated aqueous layer and the same operation was repeated twice. The aqueous layer was dehydrated and concentrated for 30 minutes at 8 kPa and an oil bath temperature of 150°C to give 80 g of 1,3-butylene glycol. According to a GC analysis, neither the peak A nor the peak B was detected (It was below the detection limit). A GC chart of 1,3-butylene glycol prepared in Example 1 is shown in Fig. 1.

### Example 2

MIBK (1 kg) and a mixture of 1 kg of water and 1 kg of 1, 3-butylene glycol manufactured by Kyowa Hakko Kogyo were sent into a packed tower made of glass (inner diameter: 27 mm; length: 500 mm) charged with Raschig rings made of glass (inner diameter: 5mm; length: 5mm; thickness: 1 mm) at the rate of 100 ml/hour from the bottom and the top of the tower, respectively. A boundary was formed near the bottom of the tower between an aqueous layer and an organic layer. The aqueous layer and the organic layer were taken out from the bottom and the top of the tower, respectively so as not to change the position of the boundary. Inner temperature of the tower at that time was 25°C. The aqueous layer obtained from the bottom of the tower was dehydrated and concentrated for 30 minutes at 8 kPa and the oil bath temperature of 150°C to give 0.95 kg of 1, 3-butylene glycol. According to a GC analysis, the sum of the area values of the peak A and the peak B to the area value of the peak of 1,3-butylene glycol was 0.0041%. The sum of the area values of the peak A and the peak B to the sum of area values of the peaks appearing during the relative retention time of 0 to 1.85 was 0.0041% when the retention time of 1,3-butylene glycol was defined as 1.

### Example 3

Warm water of 50°C was flown in a jacket of a packed tower (inner diameter: 27 mm; length: 500 mm) made of glass equipped with a jacket into which Raschig rings made of glass (inner diameter: 5 mm; length: 5 mm; thickness: 1 mm) were charged. MIBK (1 kg), 1 kg of water and 1 kg of 1,3-butylene glycol made by Kyowa Hakko Kogyo were poured into the packed tower from the bottom, the top and the place which was 10 cm beneath the top, respectively at the flowing rate of 100 ml/hour each. A boundary was formed near the bottom of the tower between an aqueous layer and an organic layer The aqueous layer and the organic layer were taken out from the bottom and the top of the tower, respectively so as not to change the position of the boundary. Inner temperature of the tower at that time was 50°C. The aqueous layer obtained from the bottom of the tower was dehydrated and concentrated for 30 minutes at 8 kPa and the oil bath temperature of 150°C to give 0.99 kg of 1,3-butylene glycol. According to a GC analysis, the sum of the area values of the peak A and the peak B to the area value of the peak of 1,3-butylene glycol was 0.0051%. The sum of the area values of the peak A and the peak B to the sum of area values of the peaks appearing during the relative retention time of 0 to 1.85 was 0.0051% when the retention time of 1,3-butylene glycol was defined as 1.

### Comparative Example 1

1,3-Butylene glycol manufactured by Kyowa Hakko Kogyo was analyzed by means of a gas chromatography. According to the GC analysis of the 1, 3-butylene glycol manufactured by Kyowa Hakko Kogyo, the sum of area values of the peak A and the peak B (the peak of 1,3-butylene glycol, the peak A and the peak B correspond to the peaks of 8.609 minutes, 12.619 minutes and 15.065 minutes, respectively) to the area value of the peak of 1, 3-butylene glycol was 0.0436%. The sum of the area values of the peak A and the peak B to the sum of area values of the peaks appearing during the relative retention time of 0 to 1.85 was 0.0436% when the retention time of 1,3-butylene glycol was defined as 1.

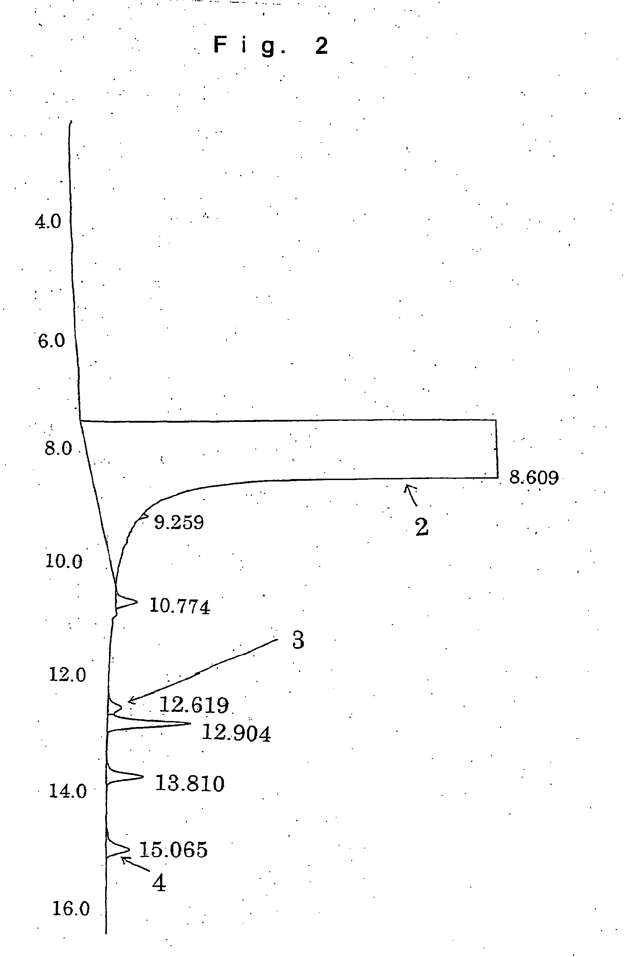

A GC analysis chart of 1,3-butylene glycol used in Comparative Example 1 is shown in Fig. 2.

When the compounds corresponding to the peak A and the peak B were analyzed by GC-MASS and CG-IR, the results were that the compounds corresponding to the peak A and the peak Bwas 2-butoxy-6-methyl-4-tetrahydropyranol as shown below(the two peaks correspond to two isomers).

GC-MASS (chemical ionization method):
Peak A: 189 (M⁺¹); peak B: 189 (M⁺¹)
GC-IR (cm⁻¹):
Peak A: 3671 (O-H), 3601 (O-H), 1145 (C-O-C)
Peak B: 3672 (O-H), 3595 (O-H), 1144 (C-O-C)

### Industrial Applicability

In accordance with the present invention, 1,3-butylene glycol having little smell is provided.

## Claims

1. 1,3-Butylene glycol where, in a gas chromatographic analysis under a specific condition, the sum of area values of peaks appearing during the specific relative retention time to the area value of the peak of 1,3-butylene glycol is not more than 0.025%.

2. 1,3-Butylene glycol where, in a gas chromatographic analysis under a specific condition, the sum of area values of peaks of compounds having a hydroxyl group and an ether bond and having a molecular weight of 188 to the area value of the peak of 1,3-butylene glycol is not more than 0.025%.

3. 1,3-Butylene glycol where, in a gas chromatographic analysis under a specific condition, the sum of area values of peaks of 2-butoxy-6-methyl-4-tetrahydropyranol to the area value of the peak of 1, 3-butylene glycol is not more than 0.025%.

4. 1,3-Butylene glycol where, in a gas chromatographic analysis under a specific condition, the sum of area values of peaks appearing during the specific relative retention time to the sum of area values of the peaks appearing during the relative retention time of 0 to 1.85 when the retention time of 1, 3-butylene glycol is defined as 1 is not more than 0-025%.

5. The 1,3-butylene glycol according to claim 1 or 4, wherein the peaks appearing during the specific relative retention time are the peaks having an apex during the relative retention time of 1.4 to 1. 48 or during the relative retention time of 1.7 to 1.8 when the retention time of 1,3-butylene glycol is defined as 1.

6. The 1,3-butylene glycol according to any of claims 1 to 5, wherein the gas chromatographic analysis under a specific condition is a gas chromatographic analysis using a column using polyethylene glycol in a liquid phase.

7. The 1,3-butylene glycol according to any of claims 1 to 6, wherein the gas chromatographic analysis under a specific condition is carried out under the following gas chromatographic analysis condition.
Condition for the gas chromatographic analysis:
Apparatus: HP 5890 Series II manufactured by Hewlett-Packard
Column: Capillary column DB-WAX (length: 30 m; inner diameter: 0.25 mm; membrane thickness: 0.25 µm) manufactured by J & W Scientific
Temperature-rising condition: After the retention at 130°C for 10 minutes, the temperature is raised up to 140°C at the rate of 1°C/minute and retained at 140°C.
Sample-introducing part: Split sample introduction method; temperature: 250°C
Flow rate of gas at the split (carrier gas: nitrogen): 67 ml/minute
Flow rate of gas through the column (carrier gas: nitrogen): 0.86 ml/minute
Detector: hydrogen flame ionization detector (FID); temperature: 250°C

8. A process for producing 1,3-butylene glycol comprising a step of mixing crude 1, 3-butylene glycol with water and an organic solvent followed by separation of an aqueous layer and an organic layer to obtain an aqueous layer containing 1,3-butylene glycol.

9. The process for producing 1,3-butylene glycol according to claim 8, wherein the aqueous layer containing 1,3-butylene glycol is subjected to distillation.
